(19) Europäisches Patentamt European Patent Office Office européen des brevets

(11) **EP 2 328 711 B1**

(12) **EUROPÄISCHE PATENTSCHRIFT**

(45) Veröffentlichungstag und Bekanntmachung des Hinweises auf die Patenterteilung:
**24.09.2014 Patentblatt 2014/39**

(21) Anmeldenummer: **08785759.5**

(22) Anmeldetag: **29.08.2008**

(51) Int Cl.:
*B23K 26/03* (2006.01)    *B23K 26/36* (2014.01)
*B23K 26/40* (2014.01)    *A61F 9/008* (2006.01)
*G01B 9/02* (2006.01)

(86) Internationale Anmeldenummer:
**PCT/EP2008/007102**

(87) Internationale Veröffentlichungsnummer:
**WO 2010/022754 (04.03.2010 Gazette 2010/09)**

(54) **VERFAHREN UND VORRICHTUNG ZUR KALIBRIERUNG DER PULSENERGIE EINER LASEREINRICHTUNG UNTER VERWENDUNG EINER KOHÄRENZOPTISCHEN INTERFEROMETRISCHEN MESSVORRICHTUNG**

METHOD OF AND APPARATUS FOR CALIBRATING THE PULSE ENERGY OF A LASER DEVICE USING A COHERENCE-OPTICAL INTERFEROMETRIC MEASURING APPARATUS

PROCEDE ET APPAREIL POUR ETALONNER L'ENERGIE D'IMPULSION D'UN DISPOSITIF LASER A L'AIDE D'UN DISPOSITIF DE MESURE INTERFEROMETRIQUE A OPTIQUE DE COHERENCE

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(43) Veröffentlichungstag der Anmeldung:
**08.06.2011 Patentblatt 2011/23**

(73) Patentinhaber: **WaveLight GmbH**
**91058 Erlangen (DE)**

(72) Erfinder:
• **RIEDEL, Peter**
**90489 Nürnberg (DE)**
• **DONITZKY, Christof**
**90542 Eckental (DE)**

(74) Vertreter: **Katérle, Axel**
**Wuesthoff & Wuesthoff**
**Patent- und Rechtsanwälte**
**Schweigerstraße 2**
**81541 München (DE)**

(56) Entgegenhaltungen:
GB-A- 2 355 310    US-A- 5 520 679
US-A1- 2003 016 353    US-A1- 2004 147 910
US-A1- 2006 100 613

• **WONG B J F ET AL: "SURFACE CHARACTERIZATION OF LASER ABLATED HARD TISSUE: A COMPARISON OF SCANNING WHITE LIGHT INTERFEROMETRY AND ELECTRON MICROSCOPY" PROCEEDINGS OF THE SPIE, SPIE, BELLINGHAM, VA, US, Bd. 2390, 1. Januar 1995 (1995-01-01), Seiten 68-75, XP009003998 ISSN: 0277-786X**

EP 2 328 711 B1

## Beschreibung

Die Erfindung betrifft ein Verfahren zur Kalibrierung der Pulsenergie einer gepulste Arbeitslaserstrahlung bereitstellenden Lasereinrichtung. Bei dem Verfahren werden mittels der Arbeitslaserstrahlung mehrere Testablationen, insbesondere Vielfachpuls-Testablationen, an einem oder mehreren Testobjekten mit jeweils unterschiedlicher Pulsenergie vorgenommen. Es wird die Ablationstiefe jeder der Testablationen gemessen und auf Grundlage der gemessenen Ablationstiefen und einer vorgegebenen Soll-Ablationstiefe eine zugehörige Soll-Pulsenergie ermittelt. Diese Soll-Pulsenergie wird an der Lasereinrichtung eingestellt siehe US 2004/147910 A).

Ferner betrifft die vorliegende Erfindung eine Lasereinrichtung zur Durchführung einer Energiekalibrierung eines Arbeitslasers, gemäß dem Oberbegriff des Anspruchs 5.

Das oben beschriebene Verfahren sowie die genannte Lasereinrichtung werden unter anderem in der refraktiven ophthalmologischen Laserchirurgie eingesetzt.

Unter refraktiver Laserchirurgie sei hier die Änderung der Abbildungseigenschaften des optischen Systems "Auge" mittels Laserstrahlung verstanden. Die Wechselwirkung der eingestrahlten Laserstrahlung mit dem Auge ändert die Brechungseigenschaften einer oder mehrerer Komponenten des Auges. Da für die Abbildungseigenschaften des Auges die Hornhaut (Kornea) maßgeblich ist, beinhaltet die Laserchirurgie des Auges in vielen Fällen eine Bearbeitung der Kornea. Durch gezielten Materialabtrag (Materialablation) wird dabei eine Formänderung der Kornea bewirkt. Man spricht deshalb auch von einer Neuformung der Kornea.

Ein prominentes Beispiel der refraktiven ophthalmologischen Chirurgie ist die LASIK (LASer-In-Situ-Keratomileusis), bei der Korneagewebe abgetragen (ablatiert) wird, um die Kornea zur Korrektur von Sehfehlern neu zu formen. Zur Ablation von Korneagewebe wird in der Regel ein Excimerlaser im UV-Bereich (typischerweise 193 nm) eingesetzt. Die Laserstrahlung wird bezüglich Zeit und Ort so über das Auge geführt, dass an ausgewählten Stellen der Kornea eine bestimmte Menge Gewebe ablatiert wird. Diese Ablation wird durch das so genannte Ablationsprofil beschrieben, d.h. das Ablationsprofil gibt die an jeder Stelle der Kornea vorzunehmende Ablation an.

Das Ablationsprofil wird in der Regel vor Durchführung des chirurgischen Eingriffs für das zu korrigierende Auge berechnet. Grundlage dieser Berechnung ist eine Vermessung des Auges in seinem Ist-Zustand. Für diese Vermessung des Auges kennt der Stand der Technik unterschiedliche Techniken, insbesondere Topografiemessgeräte (so genannte Topolyzer), Wellenfrontanalysatoren, Scheimpfluggeräte, Pachymeter sowie die subjektive Refraktionsbestimmung.

Das Ablationsprofil wird so berechnet, dass nach der Operation die Kornea eine für das behandelte Auge optimale Form hat und zuvor vorhandene optischen Abbildungsfehler des Auges weitest möglich korrigiert sind. Für die Berechnung des Ablationsprofil stehen der Fachwelt seit längerem geeignete Methoden zur Verfügung.

Ist für das zu behandelnde Auge das Ablationsprofil bestimmt, wird anschließend berechnet, wie der gewünschte Abtrag mit der zur Verfügung stehenden Laserstrahlung am besten erzielt werden kann. Dazu muss eine Beziehung zwischen der Energiedichte des Laserpulses und dem damit bewirkten Materialabtrag gefunden und beachtet werden. Diese Beziehung bildet die Grundlage für eine Kalibrierung des Arbeitslasers für ein zu bearbeitendes Material und für einen definierten Abtrag von Material. Neben der Energiedichte des Laserpulses beeinflussen eine Reihe weiterer Parameter die Höhe des Materialabtrags, wie beispielsweise die Materialeigenschaften selbst, die Materialtemperatur, die Oberflächengestaltung, etc., vorliegend soll aber primär eine Variation der Pulsenergie betrachtet werden. Das Steuerprogramm für die Laserpulse wird unter der Annahme eines bestimmten, gleichbleibenden Abtrags pro Puls berechnet. Daher ist es wichtig, dass stets diejenige Energie am Lasersystem eingestellt wird, die genau diesen angenommenen/geforderten Abtrag bewirkt.

In der ophthalmologischen Chirurgie sind verschieden ausgestaltete Methoden zur Energiekalibrierung von Lasern im Hinblick auf die Wirkung des gepulsten Laserlichts mit der zu bearbeitenden Materie bekannt:

Bei einer ersten Methode wird mittels des zu kalibrierenden Laserstrahls auf einer speziellen Kondensatorfolie eine Ablation vorgenommen. Die Ablation ruft einen Farbumschlag hervor, der dann als Maß für die Energie dient, die auf die Folie zur Ablation aufgebracht worden ist.

Bei einer weiteren Methode zur Energiekalibrierung wird auf eine Probe aus Polymethylmethacrylat (PMMA) eine refraktive Ablation aufgebracht. Anschließend wird die Änderung in der Refraktion an der Ablationsstelle mit einem Scheitelbrechwertmessgerät ermittelt.

Weiterhin wurde ein Verfahren entwickelt, bei dem auf einer PMMA-Fluenztestscheibe eine definierte Testablation vorgenommen wird. Unter Fluenz wird hier die Energie des Laserstrahls pro Fläche verstanden. Die Ablationstiefe der Testablation wird mittels eines mechanischen Tasters gemessen. Liegt die ermittelte Ablationstiefe in einem vorgegebenen Bereich, ist die Energie des Lasersystems korrekt eingestellt.

Die vorgenannten Verfahren und die zugehörigen Systeme weisen jedoch verschiedene Nachteile auf. Bei Verwendung der oben beschriebenen mechanischen Vermessung einer Fluenztestscheibe aus PMMA beispielsweise muss der Anwender nach jeder Ablation und Vermessung entscheiden, ob die eingestellte Energie nachgeregelt werden muss oder ob sie bereits korrekt eingestellt ist. Um den angestrebten Sollwert für die Energie des Laserstrahls zu erreichen, kann es vorkommen, dass der Anwender diese Prozedur mehrmals wiederholen muss. Dies ist für den Anwender teilweise kompliziert, da er sich schrittweise durch Erhöhen oder Verringern der Strahlenergie an die optimale Ablationstiefe und damit an die gewünschte Strahlenergie "herantasten" muss.

Es ist eine Aufgabe der vorliegenden Erfindung, ein verbessertes Energiekalibrierverfahren sowie eine entsprechende Lasereinrichtung anzugeben, die die genannten Nachteile vermeiden.

Ein Verfahren und eine Vorrichtung gemäß der Erfindung sind in Ansprüche 1 und 5 definiert.

Die berührungslose Ermittlung der Ablationstiefe und die Integrationsfähigkeit des Messverfahrens erlauben einen völlig neuen Ansatz bei der Ermittlung der Ablationstiefe am Testobjekt. Es kann so zum einen die Kalibrierung der Pulsenergie dahingehend verbessert werden, dass beispielsweise direkt nach der Testablation das Testobjekt vermessen wird, ohne dass das Testobjekt dazu bewegt werden muss, und so eine Automatisierung des Kalibrierungsvorgangs erzielt werden kann. Ferner wird die Genauigkeit der Kalibrierung verbessert, da mittels der kohärenzoptischen interferometrischen Messvorrichtung gleichzeitig zur Ablationstiefe die relative Position des Testobjekts zur Arbeitsebene der Lasereinrichtung ermittelt und eingestellt werden kann. Dies erlaubt eine genauere, weil der späteren Einsatzsituation besser angepasste Ermittlung der Pulsenergie des Arbeitslasers.

Gemäß einer vorteilhaften Ausführungsform des Verfahrens arbeitet die Messvorrichtung nach dem Prinzip der OLCR ("OLCR = Optical Low Coherence Reflectometrie"/optische Kurzkohärenzreflektometrie). Dieses Messprinzip wird in der Pachymetrie bei der Vermessung der Dicke der Kornea eingesetzt. Die Erfindung lehrt somit, ein für die Dickenmessung der Kornea geeignetes Messverfahren für die Messung der Ablationstiefe einer Testablation an dem Testobjekt zu verwenden. Somit kann eine Veränderung der refraktiven Eigenschaften des Testobjekts, hervorgerufen durch die Ablation, genau und reproduzierbar bestimmt werden.

Eine Integration der interferometrischen Messvorrichtung ist gemäß der Erfindung einfach durchzuführen, wenn die Ablationstiefen mittels eines zur Arbeitslaserstrahlung gleichachsig verlaufenden Messstrahls der Messeinrichtung vermessen werden. Es können bereits vorhandene optische Einrichtungen, wie z.B. Spiegel, Linsen, etc., die zur Führung und Formung des Arbeitslaserstrahls dienen, gleichzeitig für den Messstrahl der Messeinrichtung verwendet wenden. Für eine Automatisierung des Energiekalibriervorgangs von den gemäß der Erfindung mehrere der Testablationen an demselben Testobjekt vorgenommen und dieses zwischen aufeinander folgenden Testablationen relativ zu der Messeinrichtung bewegt werden. Insbesondere kann vorgesehen sein, dass das Testobjekt nicht manuell, sondern motorisiert bewegt wird, so dass ohne einen Eingriff eines Anwenders eine Serie von Testablationen an dem Testobjekt durchgeführt werden kann.

Gemäß der Erfindung ist vorgesehen, dass die Testablationen in radialem Abstand von der Scheibenmitte einer als Testobjekt verwendeten Testscheibe angebracht werden und ein die Testscheibe tragender Drehteller zwischen aufeinander folgenden Testablationen um einen vorgegebenen Drehwinkel gedreht wird. Die Testablationen sind somit gleichmäßig mit maximalem Abstand zueinander auf der Testscheibe verteilt und es wird eine Bewegung des Testobjekts in technisch einfacher Weise realisiert. Eine Verfälschung des Messergebnisses durch einen Materialniederschlag benachbarter Ablationen wird verringert bzw. vermieden.

Ferner kann bei einem erfindungsgemäßen Verfahren vorgesehen sein, dass an einem Testobjekt, insbesondere einem mit wenigstens einer Testablation versehenen Testobjekt, eine Kontrollablation mit der ermittelten Soll-Pulsenergie vorgenommen und die Ablationstiefe der Kontrollablation anschließend mit der Messvorrichtung gemessen wird. Dies ist insbesondere nach einer Serie von Testablationen an dem Testobjekt sinnvoll. Nach Ermittlung der Ablationstiefen der mehreren Testablationen kann beispielsweise unter Annahme eines funktionalen Zusammenhangs zwischen aufgebrachter Pulsenergie und Ablationstiefe eine mit der Soll-Ablationstiefe korrelierende Soll-Pulsenergie abgeleitet werden. Durch Vornahme einer Kontrollablation mit der ermittelten Soll-Pulsenergie kann verifiziert werden, ob diese Soll-Pulsenergie zu der gewünschten Soll-Ablationstiefe führt.

Zur Kühlung des Testobjekts bzw. der Testobjekte oder/und zur Reinhaltung des Bereichs über den Testablationen empfiehlt es sich, einen Luftstrom zu erzeugen und zumindest auf den ablatierten Teil eines Testobjekts zu lenken. Dies kann bereits während der Durchführung einer Testablation oder zumindest danach geschehen, um für eine präzise, optische Vermessung der Ablationskrater etwaige Ablationsprodukte in den unvermeidlichen Ablationswolken wegzublasen.

Neben dem beschriebenen erfindungsgemäßen Verfahren zur Pulsenergiekalibrierung findet der Erfindungsgedanke auch in einer Lasereinrichtung Niederschlag, siehe Anspruch 5.

Die Erfindung wird nachfolgend anhand der beigefügten Zeichnungen weiter erläutert. Es stellen dar:

Fig. 1 ein Ablaufdiagramm eines erfindungsgemäßen Ausführungsbeispiels für ein Verfahren zur Energiekalibrierung einer Lasereinrichtung,

Fig. 2 ein Energie-Ablationstiefe-Diagramm für verschiedene durchgeführte Testablationen,

Fig. 3a und 3b in schematischer Blockdarstellung eine erfindungsgemäße Ausführungsform einer Lasereinrichtung vor und nach einer Testablation,

Fig. 4a und 4b ein Positioniermodul zur definiert positionsveränderbaren Halterung eines Testobjekts in Drauf- bzw. Seitenansicht, sowie

Fig. 5 ein mit Testablationsstellen und einer Kontrollablationsstelle versehenes Testobjekt in Draufsicht.

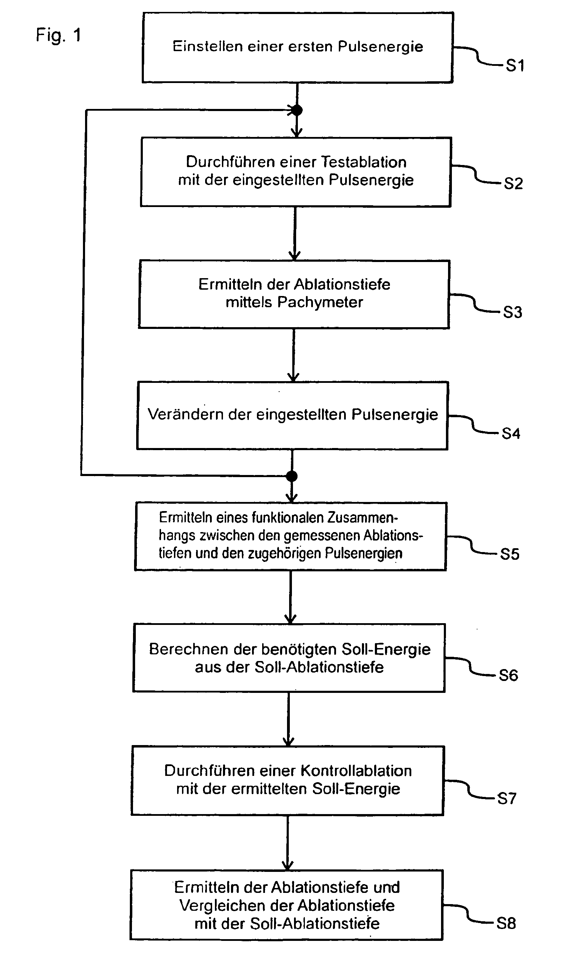

In Fig. 1 ist eine mögliche Ausführungsform eines erfindungsgemäßen Verfahrens mit den Schritten S1-S8 dargestellt. Zur Kalibrierung der Pulsenergie einer gepulste Laserstrahlung bereitstellenden Lasereinrichtung wird zunächst an der Lasereinrichtung eine erste Pulsenergie eingestellt (S1). Dieser erste Wert der Pulsenergie kann entweder in der Nähe der gewünschten Sollenergie liegen oder an einem Rand eines Bereichs liegen, der die gewünschte Pulsenergie abdeckt.

Mit dieser ersten eingestellten Pulsenergie wird eine Testablation an einem Testobjekt durchgeführt, z.B. mit mehreren Tausend Laserpulsen (S2). Dabei wird an der Testablationsstelle durch die auftreffende Pulsenergie des Arbeitslaserstrahls eine bestimmte Menge des Testobjektmaterials entfernt, so dass sich eine Vertiefung auf dem Testobjekt ausbildet.

Diese Ablationstiefe wird mittels eines Pachymeters ermittelt (S3). Mit der Pachymetrie ist es möglich, den Abstand des Testobjekts von der Lasereinrichtung exakt einzustellen und gleichzeitig die Dicke des Testobjekts zu messen. Ein geeignetes Material für das Testobjekt sollte bei der Ablation entweder eine Wechselwirkung mit dem Arbeitslaserstrahl aufweisen, die der zwischen dem späteren Zielobjekt (z.B. der Kornea) und dem Arbeitslaserstrahl vergleichbar ist. Oder es wird ein Material verwendet, dessen Reaktion auf die eingestrahlten Laserpulse zwar unterschiedlich (z.B. unterschiedlich stark) im Vergleich zu dem später zu behandelnden Material (z.B. biologisches Gewebe) ist, wobei aber der Zusammenhang zwischen der Reaktion des Testmaterials und der Reaktion des zu behandelnden Materials zumindest in grober Näherung bekannt ist, etwa durch empirische Daten. Ferner muss das Material des Testobjekts einer Pachymetrie zugänglich sein. Diesbezüglich hat sich das Material Polymethylmetacrylat (PMMA) in Form dünner Plättchen als besonders geeignet herausgestellt. Nach dem Ermitteln der Tiefe der Ablationsstelle existiert ein erstes Ablationstiefe-Pulsenergie-Wertepaar.

Ausgehend von diesem Messwert wird die Pulsenergie für eine weitere Testablation verändert (S4). Zu diesem Zweck wird ausgehend von der ersten Pulsenergie die nächste Pulsenergie um eine Schrittweite erhöht/verringert. Alternativ zu diesem schrittweisen Abtasten eines Energiebereichs sind auch andere Verfahren denkbar. Beispielsweise könnte der Soll-Energiewert auch durch ein iteratives Intervallschachtelungsverfahren ermittelt werden.

Das Verfahren setzt sich unter erneuter Durchführung der Schritte S2-S4 fort, bis entweder der vorher festgelegte Pulsenergiebereich durchlaufen oder das Testobjekt mit der maximalen Anzahl an Testablationen versehen ist.

Daran anschließend wird zwischen den gemessenen Ablationstiefen und den zugehörigen Pulsenergien ein Zusammenhang ermittelt (S5). Dabei kann beispielsweise von einer linearen Korrelation der Ablationstiefe von der Pulsenergie ausgegangen werden und den Pulsenergie-Ablationstiefe-Wertepaaren eine Ausgleichsgerade angepasst werden ("linearer Fit"). Gegebenenfalls können aber auch Regressionsanalysen höherer Ordnung oder Anpassungen nach anderen Modellen vorgenommen werden.

Aus dem so ermittelten funktionalen Zusammenhang kann die benötigte Sollenergie aus der gewünschten Soll-Ablationstiefe berechnet werden (S6).

Der Soll-Energiewert wird eingestellt und es wird eine Kontrollablation mit der ermittelten Soll-Energie durchgeführt (S7). Die danach ermittelte Ablationstiefe der Kontrollablation sollte nun der gewünschten Soll-Ablationstiefe entsprechen, was durch ein Vergleichen der Kontrollablationstiefe mit der Soll-Ablationstiefe verifiziert wird (S8).

Auf diese Weise ist der Arbeitslaser nach Durchführen des erfindungsgemäßen Verfahrens auf einfache und genaue Weise hinsichtlich seiner Ablationswirkung kalibriert und beispielsweise für das Durchführen einer ophthalmologischen Laserchirurgie vorbereitet.

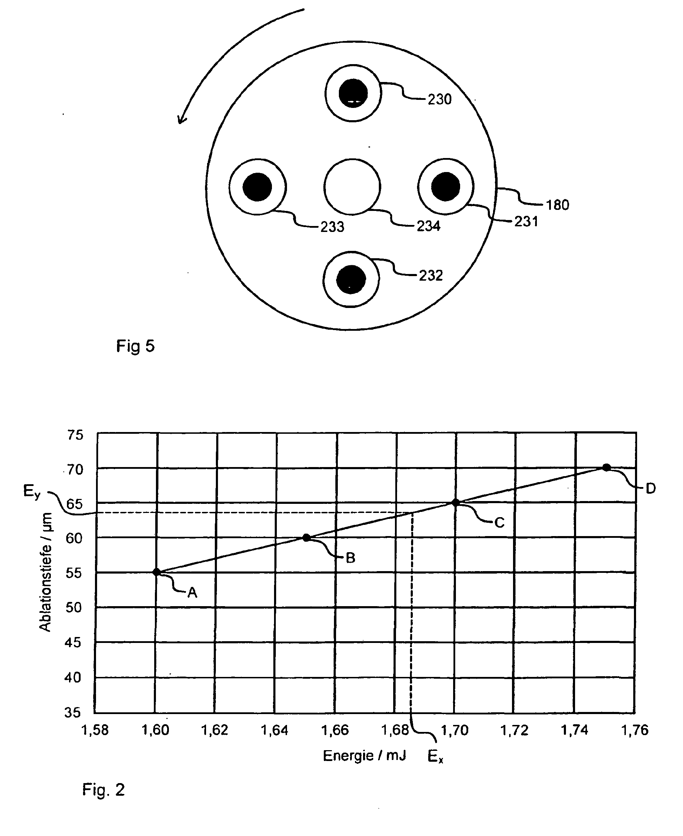

Fig. 2 zeigt ein Beispiel eines derartigen Kalibriervorgangs gemäß dem erfindungsgemäßen Verfahren. In dem Diagramm ist an der Abszisse die Energie eines Laserpulses in mJ aufgetragen, es ist ein Bereich von 1,58-1,76 mJ abgebildet. Es wurden Testablationen mit den Energiewerten 1,60 mJ, 1,65 mJ, 1,70 mJ und 1,75 mJ vorgenommen. Die mittels Pachymetrie ermittelten Ablationstiefen sind an der Ordinate in $\mu$m aufgetragen, in einem Bereich von 35-75 $\mu$m. Die den Pulsenergiewerten zugeordneten Ablationstiefen sind 55 $\mu$m, 60 $\mu$m, 65 $\mu$m und 70 $\mu$m. An diese vier gemessenen Wertepaare A-D lässt sich folgende Ausgleichsgerade anpassen:

$$y = 100x - 105$$

Dabei steht der Parameter y für die Ablationstiefe in $\mu$m und der Parameter x für die Energie des Laserpulses in mJ. Beträgt nun beispielsweise die Soll-Ablationstiefe 63,5 $\mu$m, was in dem Diagramm mit dem Symbol Ey bezeichnet ist, ergibt sich für die vorgeschlagene Sollenergie 1,658 mJ. Dieser Wert ist in dem Diagramm mit dem Symbol $E_x$ bezeichnet.

Selbstverständlich ist die Verwendung einer linearen Regression nur eine von vielen Möglichkeiten, um aus den gemessenen Wertepaaren die gewünschte Sollenergie für eine vorgegebene Ablations-Solltiefe zu ermitteln.

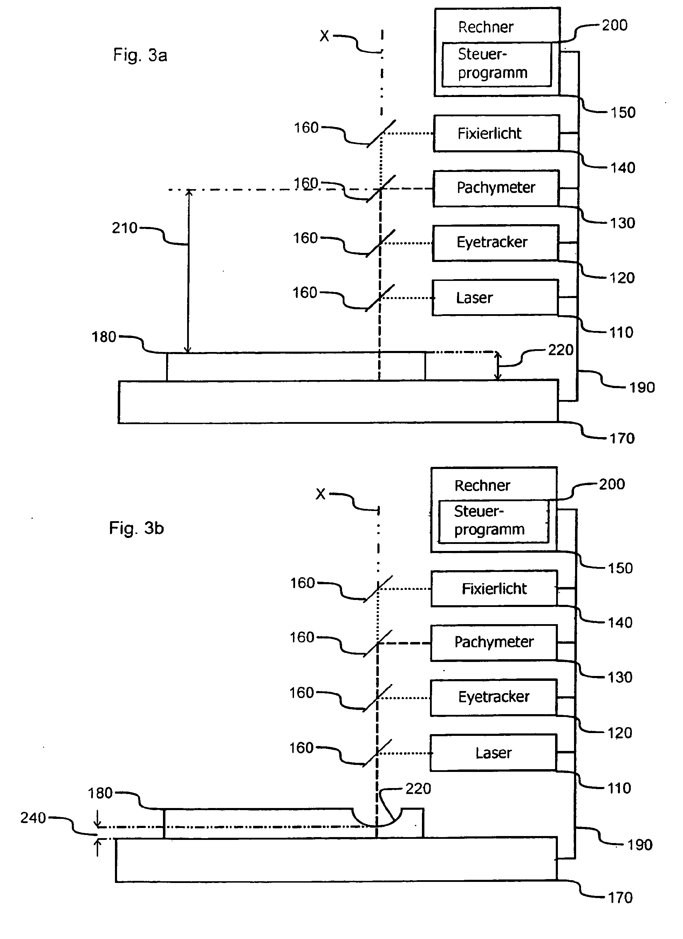

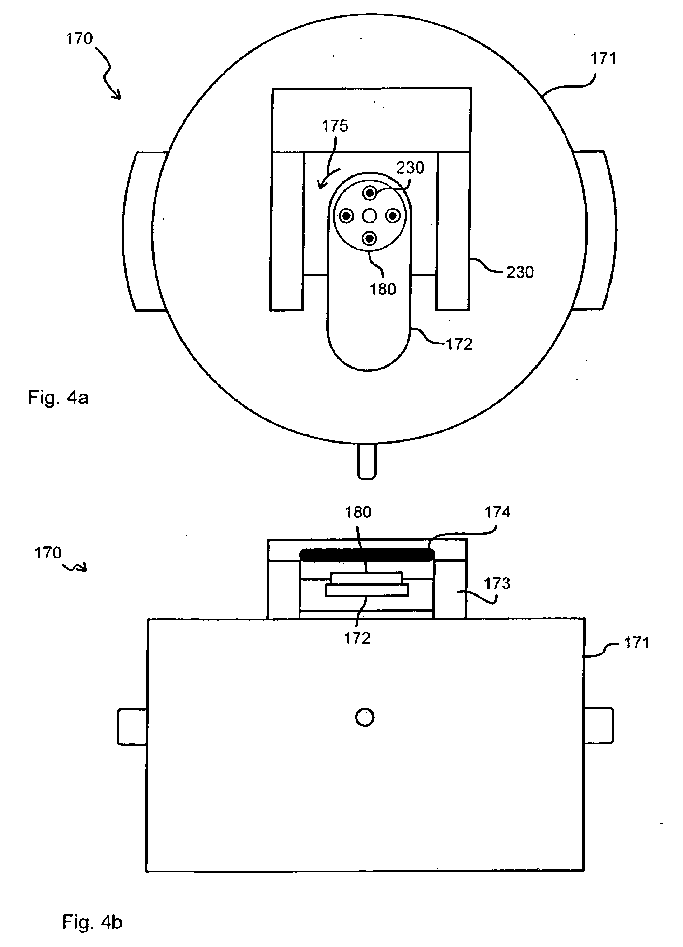

Die in den Figuren 3a und 3b dargestellte Lasereinrichtung 100 ist beispielsweise zur ophthalmologischen Laserchirurgie ausgelegt und umfasst als Laserstrahlungsquelle einen für Kornea-Ablationen geeigneten Arbeitslaser 110, der gepulste Strahlung abgibt, einen Eyetracker 120, der zur Nachverfolgung der Augenbewegungen während der Kornea-Behandlung dient, sowie ein Fixierlicht 140, das während der Kornea-Behandlung von dem Patienten zu fixieren ist, um die Augenbewegungen möglichst gering zu halten. Ferner ist in die Lasereinrichtung 100 ein Pachymeter 130 integriert, das zur Erfassung der Korneadicke mittels OLCR ("Optical Low Coherence Reflectometrie"/optische Kurzkohärenzreflektometrie) geeignet ist. Alle genannten Komponenten 110-140 arbeiten auf einer gemeinsamen optischen Achse X, die durch verschiedene optische Komponenten, wie etwa Spiegel, Linsen, etc. realisiert wird. Diese optische Komponenten sind in den Figuren 3a und 3b lediglich schematisch als Spiegel 160 angedeutet. Neben den genannten und an sich vorbekannten Komponenten weist die erfindungsgemäße Lasereinrichtung ein Positioniermodul 170 sowie einen Rechner 150 auf. Das Positioniermodul 170 ist lediglich schematisch als Block dargestellt, eine detailliertere Beschreibung desselben findet sich im Zusammenhang mit den Figuren 4a und 4b. Auf dem Positioniermodul auswechselbar angebracht ist eine sogenannten Fluenztestscheibe 180 aus Polymethylmetacrylat (PMMA). Sowohl die Komponenten 110-140 als auch das Positioniermodul 170 sind über Steuerungsleitungen 190 mit dem Rechner 150 verbunden. Der Rechner weist ein Steuerprogramm 200 auf, das zur Steuerung der Komponenten 110-140 sowie des Positioniermoduls 170 ausgelegt ist.

Zur Ermittlung einer Soll-Pulsenergie der Lasereinrichtung 100 werden sowohl der Abstand der Fluenztestscheibe 180 von der Lasereinrichtung 100, insbesondere von dem Pachymeter 130, dargestellt durch den Doppelpfeil 210, als auch die Dicke der Fluenztestscheibe 180, dargestellt durch einen weiteren Doppelpfeil 220, ermittelt. Ausgehend von diesen Messdaten werden der Arbeitslaser 110 und das Positioniermodul 170 so angesteuert, dass mehrere Testablationen auf verschiedenen Stellen der Fluenztestscheibe 180 vorgenommen werden. In Fig. 3b ist eine derartige Testablation 230 schematisch angedeutet. Direkt nach der Durchführung einer Testablation wird die Testablationsstelle 230 mittels des Pachymeters 130 vermessen, so dass die sich ergebende Ablationstiefe bestimmt wird. Diese ist in Fig. 3b schematisch bei Bezugszeichen 240 angedeutet. Nach Durchführen einer Reihe von Testablationen bei unterschiedlichen Pulsenergien kann, wie bereits vorstehend im Zusammenhang mit den Figuren 1 und 2 beschrieben, die gewünschte Soll-Pulsenergie für eine Zielablationstiefe ermittelt werden.

In den Figuren 4a und 4b ist das bereits im Zusammenhang mit den Figuren 3a und 3b erwähnte Positioniermodul 170 detaillierter dargestellt. Fig. 4a zeigt eine schematische Draufsicht, während Fig. 4b eine schematische Seitenansicht des Positioniermoduls abbildet. Das Positioniermodul 170 weist ein Gehäuse 171 auf. An seiner Oberseite ist eine Aufnahmeplatte 172 zur Aufnahme der Fluenztestscheibe 180 vorgesehen, wobei die Aufnahmeplatte 172 an einer Aufnahmevorrichtung 173 angeordnet ist, die fest mit dem Gehäuse 171 verbunden ist. In die Aufnahmevorrichtung 173 ist eine Luftaustrittsöffnung 174 integriert.

Die Fluenztestscheibe 180 wird von der Aufnahmeplatte 172 gehalten. Wird eine Serie von Testablationen durchgeführt, werden die Testablationsstellen 230 gleichmäßig im 90° Winkel auf der Fluenztestscheibe 180 verteilt, indem die Fluenztestscheibe zwischen den einzelnen Testablationen, beispielsweise gegen den Uhrzeigersinn, um 90° gedreht wird, z.B. mittels eines von dem Rechner gesteuerten motorischen Antriebs. Dies ist in Fig. 4a durch den Pfeil 175 angedeutet. Zur Gewährleistung reproduzierbarer Messergebnisse wird auf die Oberseite der Fluenztestscheibe ein Luftstrom gerichtet, der an der Öffnung 174 austritt und über die Oberfläche der Fluenztestscheibe 180 hinwegstreicht. Der Luftstrom kann dazu dienen, etwaige Wärme abzuführen. Ein Gebläse zur Erzeugung des Luftstroms sowie ein Stellmotor zum Drehantrieb der Aufnahmeplatte 172 können beispielsweise in dem Gehäuse 171 untergebracht sein (beide nicht abgebildet). Der Luftstrom ist zweckmäßigerweise so gerichtet, dass er Ablationsprodukte in der über der Testscheibe entstehenden Ablationswolke entfernen und so den Bereich über der Scheibe sauber und frei von störenden Teilchen halten kann.

Fig. 5 stellt eine vergrößerte Ansicht einer Fluenztestscheibe 180 dar. Eine derartige kreisförmige Fluenztestscheibe kann beispielsweise aus Polymethylmetacrylat (PMMA) bestehen, einen Radius von 30 mm aufweisen und cirka 4 mm dick sein. Selbstverständlich kann eine Fluenztestscheibe in beliebigen Formen (beispielsweise quadratisch, streifenförmig, etc.) sowie mit Abmessungen gefertigt werden, die von den vorgenannten abweichen. Die Testablationsscheibe 180 weist vier periphere Ablationsstellen 230-233 auf, die hier in gleichem Abstand zueinander möglichst randnahe angeordnet sind. Zweck und Ziel dieser Anordnung ist es, eine Verschmutzung der noch zur Ablation benutzten Flächen auf der Scheibe durch Ablationsprodukte einer vorausgehenden Testablation zu verhindern, indem ein größtmöglicher Abstand der Testablationsstellen 230-233 zueinander sichergestellt wird.

Zentral in der Mitte der Fluenztestscheibe ist eine Kontrolltestablationsstelle 234 angeordnet, die nach der Vermessung der Ablationstiefe der Testablationen an den Testablationsstellen 230-233 und der Auswertung der Ablationstiefen mit einer Pulsenergie ablatiert wird, die der ermittelten Soll-Energie entspricht. Die Kontrollablationsstelle 234 kann bei Bereitstellung einer geeigneten Bewegungsmechanik ebenfalls mit dem Pachymeter 130 vermessen werden. Alternativ dazu kann aber auch eine mechanische Messmethode, wie etwa das Vermessen mittels eines Tiefenmesstasters eingesetzt werden. Durch die Verwendung zweier unabhängiger Messmethoden wird eine geringere Fehlerwahrscheinlichkeit erreicht.

**Patentansprüche**

1. Verfahren zur Kalibrierung der Pulsenergie einer gepulste Arbeitslaserstrahlung bereitstellenden Lasereinrichtung, wobei bei dem Verfahren mittels der Arbeitslaserstrahlung mehrere Testablationen, insbesondere Vielfachpuls-Testablationen, an einem oder mehreren Testobjekten mit jeweils unterschiedlicher Pulsenergie vorgenommen werden, ferner die Ablationstiefe jeder der Testablationen gemessen wird und anschließend auf Grundlage der gemessenen Ablationstiefen und einer vorgegebenen Soll-Ablationstiefe eine zugehörige Soll-Pulsenergie ermittelt und an der Lasereinrichtung eingestellt wird, **dadurch gekennzeichnet, dass** die Ablationstiefen mittels einer kohärenzoptischen interferometrischen Messvorrichtung gemessen werden, dass die Ablationstiefen mittels eines längs der Richtung der Arbeitslaserstrahlung verlaufenden Messstrahls der Messeinrichtung vermessen werden und dass mehrere der Testablationen an demselben Testobjekt vorgenommen werden und dieses zwischen aufeinanderfolgenden Testablationen relativ zu der Messeinrichtung bewegt wird, wobei die Testablationen in radialem Abstand von der Scheibenmitte einer als Testobjekt verwendeten Testscheibe angebracht werden und die Testscheibe zwischen aufeinanderfolgenden Testablationen um einen vorgegebenen Drehwinkel gedreht wird.

2. Verfahren nach Anspruch 1, **dadurch gekennzeichnet, dass** die Messvorrichtung nach dem Prinzip der OLCR arbeitet.

3. Verfahren nach einem der vorhergehenden Ansprüche, **dadurch gekennzeichnet, dass** an einem Testobjekt, insbesondere einem mit wenigstens einer der Testablationen versehenen Testobjekt, eine Kontrollablation mit der ermittelten Soll-Pulsenergie vorgenommen und die Ablationstiefe der Kontrollablation anschließend mit der Messvorrichtung gemessen wird.

4. Verfahren nach einem der vorhergehenden Ansprüche, **dadurch gekennzeichnet, dass** ein Luftstrom erzeugt und zumindest auf den ablatierten Teil eines Testobjekts gerichtet wird.

5. Lasereinrichtung, insbesondere zur Durchführung des Verfahrens nach einem der vorhergehenden Ansprüche, umfassend

- eine Laserstrahlungsquelle (110), welche gepulste Arbeitslaserstrahlung zur Bearbeitung eines Objekts bereitstellt, **gekennzeichnet durch**:
- eine kohärenzoptische interferometrische Messvorrichtung (130) zum Messen mindestens eines Längenmaßes unter Verwendung eines längs der Richtung der Arbeitslaserstrahlung verlaufenden Messstrahls,
- ein steuerbares Positioniermodul (170) zur Positionierung eines Testobjekts (180) in mehreren definierten Positionen relativ zu der Laserstrahlungsquelle (110), wobei das Positioniermodul eine Aufnahmeplatte zur Aufnahme einer als Testobjekt verwendeten Testscheibe sowie einen Stellmotor zum Drehantrieb der Aufnahmeplatte aufweist,
- einen die Laserstrahlungsquelle (110), die Messvorrichtung (130) und das Positioniermodul (170) steuernden Rechner, welcher dazu ausgelegt ist, unter der Steuerung eines Steuerprogramms (200) die folgenden Aktionen zur Kalibrierung der Laserstrahlungsquelle (110) zu bewirken:
- Durchführen mehrerer Testablationen, insbesondere Vielfachpuls-Testablationen, an demselben Testobjekt mittels der Arbeitslaserstrahlung mit jeweils unterschiedlicher Pulsenergie, wobei die Testablationen in radialem Abstand von der Scheibenmitte der als Testobjekt verwendeten Testscheibe angebracht werden,
- Steuern des Positioniermoduls, um die Testscheibe zwischen aufeinanderfolgenden Testablationen **durch** Drehung um einen vorgegebenen Drehwinkel in verschiedene der definierten Relativpositionen zur Laserstrahlungsquelle zu bewegen,
- Messen der Ablationstiefe jeder der Testablationen mittels der Messeinrichtung (130),
- Ermitteln einer Soll-Pulsenergie auf Grundlage der gemessenen Ablationstiefen und einer vorgegebenen Soll-Ablationstiefe und ggf. Einstellen der ermittelten Soll-Pulsenergie für die Arbeitslaserstrahlung.

**Claims**

1. A method of calibrating the pulse energy of a laser device providing pulsed working laser radiation, wherein in said method, by means of the working laser radiation, multiple test ablations, in particular multiple-pulse test ablations, are carried out on one or more test objects, each with different pulse energy, wherein further the ablation depth of each of the test ablations is measured, and then, on the basis of the measured ablation depths and a specified setpoint ablation depth, an associated setpoint pulse energy is determined and set on the laser device;

**characterized in that** the ablation depths are measured by means of a coherent optical interferometric measuring device, the ablation depths are measured by means of a measuring beam of the measuring device extending along the direction of the working laser radiation, and a plurality of the test ablations are carried out on the same test object, which is moved relative to the measuring device between successive test ablations, wherein the test ablations are applied at a radial distance from the disc centre of a test disc used as a test object, and wherein the test disc is turned by a specified angle of rotation between successive test ablations.

2. The method according to claim 1, **characterized in that** the measuring device operates according to the OLCR principle.

3. The method according to any one of the preceding claims, **characterized in that**, on a test object, in particular a test object provided with at least one of the test ablations, a control ablation is carried out with the determined setpoint pulse energy, and the ablation depth of the control ablation is then measured by the measuring device.

4. The method according to any one of the preceding claims, **characterized in that** an air current is generated and directed at least onto the ablated part of a test object.

5. A laser device, in particular for carrying out the method according to any one of the preceding claims, comprising:

   - a laser radiation source (110) providing pulsed working laser radiation for processing an object; **characterized by**:
   - a coherent optical interferometric measuring device (130) for measuring at least one longitudinal extension using a measuring beam extending along the direction of the working laser radiation;
   - a controllable positioning module (170) for positioning a test object (180) in a plurality of defined positions relative to the laser radiation source (110), wherein the positioning module comprises a receiving plate for receiving a test disc used as a test object and a servo motor for rotationally driving the receiving plate;
   - a computer controlling the laser radiation source (110), the measuring device (130) and the positioning module (170), and adapted for causing the following actions for calibrating the laser radiation source (110) under the control of a control program (200):
   - carrying out a plurality of test ablations, in particular multiple-pulse test ablations, on the same test object by means of the working laser radiation, with different pulse energy in each case, wherein the test ablations are applied at a radial distance from the disc centre of a test disc used as a test object;
   - controlling the positioning module for moving the test disc between successive test ablations, by turning by a specified angle of rotation, in various of the defined relative positions to the laser radiation source;
   - measuring the ablation depth of each of the test ablations by means of the measuring device (130),
   - determining a setpoint pulse energy on the basis of the measured ablation depths and a specified setpoint ablation depth, and, if appropriate, setting the determined setpoint pulse energy for the working laser radiation.

## Revendications

1. Procédé pour étalonner l'énergie d'impulsion d'un dispositif laser qui génère un rayonnement laser de travail pulsé, ce rayonnement laser de travail servant pendant ledit procédé à effectuer plusieurs ablations d'essai, en particulier des ablations d'essai par impulsions multiples, sur un ou plusieurs objets d'essai, avec une énergie d'impulsion à chaque fois différente, la profondeur d'ablation de chacune des ablations d'essai réalisées étant en outre mesurée afin de déterminer ensuite, sur la base des profondeurs d'ablation mesurées et d'une profondeur d'ablation théorique prédéfinie, une énergie d'impulsion théorique correspondante à régler sur le dispositif laser, **caractérisé en ce que** les profondeurs d'ablation sont mesurées à l'aide d'un dispositif de mesure par interférométrie optique cohérente, **en ce que** la mesure des profondeurs d'ablation s'opère au moyen d'un faisceau de mesure émis par ledit dispositif de mesure et s'étendant le long de la direction du rayonnement laser de travail et **en ce que** plusieurs des ablations d'essai réalisées sont effectuées sur le même objet d'essai, lequel est déplacé par rapport au dispositif de mesure entre les ablations d'essai successives, ces ablations d'essai étant réalisées à une distance radiale du centre d'un disque éprouvette utilisé comme objet d'essai et le disque éprouvette étant amené à pivoter selon un angle de rotation prédéfini entre les ablations d'essai successives.

2. Procédé selon la revendication 1, **caractérisé en ce que** le dispositif de mesure fonctionne selon le principe de la réflectométrie à faible cohérence (OLCR).

3. Procédé selon l'une des revendications précédentes, **caractérisé en ce qu'**une ablation de contrôle est effectuée sur un objet d'essai, en particulier sur un objet d'essai ayant déjà reçu au moins une des ablations d'essai, avec l'énergie d'impulsion théorique déterminée, et **en ce que** la profondeur de l'ablation de contrôle est ensuite mesurée à l'aide du dispositif de mesure.

4. Procédé selon l'une des revendications précédentes, **caractérisé en ce qu'**un courant d'air est généré et dirigé au moins sur la partie éliminée par ablation d'un objet d'essai.

5. Dispositif laser, destiné en particulier à mettre en oeuvre le procédé selon l'une des revendications précédentes, comprenant :

   - une source de rayonnement laser (110) qui génère un rayonnement laser de travail pulsé pour le traitement d'un objet, **caractérisé par** :
   - un dispositif de mesure par interférométrie optique cohérente (130) pour mesurer au moins une longueur au moyen d'un faisceau de mesure s'étendant le long de la direction du rayonnement laser de travail,
   - un module de positionnement (170) commandable pour positionner un objet d'essai (180) dans plusieurs positions définies par rapport à la source de rayon laser (110), ce module de positionnement présentant un plateau récepteur pour le logement du disque éprouvette utilisé comme objet d'essai et un servomoteur pour l'entraînement en rotation du plateau récepteur,
   - un ordinateur commandant la source de rayonnement laser (110), le dispositif de mesure (130) et le module de positionnement (170), lequel ordinateur est conçu pour faire se dérouler, sous la commande d'un programme de commande (200), les actions suivantes en vue de l'étalonnage de la source de rayonnement laser (110) :
   - la réalisation de plusieurs ablations d'essai, en particulier des ablations d'essai par impulsions multiples, sur le même objet d'essai au moyen du rayonnement laser de travail avec une énergie d'impulsion à chaque fois différente, les ablations d'essai étant réalisées à une distance radiale du centre d'un disque éprouvette utilisé comme objet d'essai,
   - la commande du module de positionnement pour déplacer le disque éprouvette entre les ablations d'essai successives et l'amener par rotation selon un angle de rotation prédéfini dans une des différentes positions définies par rapport à la source de rayonnement laser,
   - la mesure de la profondeur d'ablation de chacune des ablations d'essai au moyen du dispositif de mesure (130),
   - la détermination d'une énergie d'impulsion théorique sur la base des profondeurs d'ablation mesurées et d'une profondeur d'ablation théorique prédéfinie, et le réglage éventuel de l'énergie d'impulsion théorique déterminée pour le rayonnement laser de travail.

Fig. 1

Einstellen einer ersten Pulsenergie — S1

Durchführen einer Testablation
mit der eingestellten Pulsenergie — S2

Ermitteln der Ablationstiefe
mittels Pachymeter — S3

Verändern der eingestellten Pulsenergie — S4

Ermitteln eines funktionalen Zusammenhangs zwischen den gemessenen Ablationstiefen und den zugehörigen Pulsenergien — S5

Berechnen der benötigten Soll-Energie
aus der Soll-Ablationstiefe — S6

Durchführen einer Kontrollablation
mit der ermittelten Soll-Energie — S7

Ermitteln der Ablationstiefe und
Vergleichen der Ablationstiefe
mit der Soll-Ablationstiefe — S8

Fig. 3a

X

| Rechner | —200 |
| Steuer-programm | |

—150

160 —— Fixierlicht —140

160 —— Pachymeter —130

210 —— Eyetracker —120

160 —— Laser —110

180 —— 220

190

170

Fig. 3b

X

| Rechner | —200 |
| Steuer-programm | |

—150

160 —— Fixierlicht —140

160 —— Pachymeter —130

160 —— Eyetracker —120

160 —— Laser —110

180 —— 220

240 ——

190

170

Fig 5

Fig. 2

Fig. 4a

Fig. 4b

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2004147910 A